(19) Europäisches Patentamt
European Patent Office
Office européen des brevets

(11) Publication number: **0 063 858**
**B1**

(12) # EUROPEAN PATENT SPECIFICATION

(45) Date of publication of patent specification: **20.03.85**

(21) Application number: **82301026.9**

(22) Date of filing: **01.03.82**

(51) Int. Cl.[4]: **C 07 K 5/06**, C 07 K 5/08, C 07 C 149/243, A 61 K 37/02, A 61 K 31/20 // C07D309/30

(54) **Leukotriene analogues, their preparation and use in pharmaceutical compositions.**

(30) Priority: **03.03.81 GB 8106695**

(43) Date of publication of application:
**03.11.82 Bulletin 82/44**

(45) Publication of the grant of the patent:
**20.03.85 Bulletin 85/12**

(84) Designated Contracting States:
**AT BE CH DE FR GB IT LI LU NL SE**

(56) References cited:

**JOURNAL OF THE AMERICAN CHEMICAL SOCIETY, vol. 102, no. 4, 13 February 1980, Gaston, COREY et al. "Sterospecific total synthesis of a 'Slow Reacting Substance' of anaphylaxis, leukotriene C-1", pages 1436 to 1439**
**TETRAHEDRON LETTERS, vol. 21, no. 36, 1980, Oxford, COREY et al. "Total synthesis of slow reacting substances (SRSs): 6-EPI-Leukotriene C and 6-EPI Leukotriene D", pages 3463 to 3466**
**TETRAHEDRON LETTERS, vol. 21, no. 42, 1980, Oxford, BAKER et al. "Synthesis, Separation and N.M.R. Spectra of three Double Bond Isomers of Leukotriene A Methyl Ester", pages 4123 to 4126**

(73) Proprietor: **Lilly Industries Limited**
**Lilly House Hanover Square**
**London W1R 0PA (GB)**

(72) Inventor: **Baker, Stephen Richard**
**Orchard Close Oaklea Drive**
**Eversley Hampshire (GB)**
Inventor: **Jamieson, William Boffey**
**"Inverkip" 31, Kettlewell Close**
**Horsell Woking Surrey (GB)**
Inventor: **Ross, William James**
**8, Keswick Drive**
**Lightwater Surrey (GB)**

(74) Representative: **Hudson, Christopher Mark et al**
**Erl Wood Manor**
**Windlesham Surrey GU20 6PH (GB)**

(56) References cited:
**Chemical Abstracts, vol. 93, no. 13, 29 September 1980, Columbus, Ohio, USA, CLARK et al. "II-trans-Leukotriene C: a naturally occurring slow reacting substance", page 490, column 1, abstract no. 130325r**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 063 858 B1**

**Description**

The present invention relates to leukotriene analogues, their production and use, and pharmaceutical preparations containing same.

A variety of leukotrienes and their analogues are described in the literature. For example, leukotriene C-1 is described by G. Corey et al. in Journal of the American Chemical Society Vol. 102, No. 4 pages 1436 to 1439 (1980) and 6-*epi*-leukotriene C and 6-*epi*-leukotriene D in Tetrahedron Letters Vol. 21, No. 36 pages 3463 to 3466 (1980). Baker et al. in Tetrahedron Letters Vol. 21, No. 42 pages 4123 to 4126 describe isomers of leukotriene A methyl ester, and Clark et al. in Biochem. Biophys. Res. Commun. Vol. 94 No. 4 pages 1133 to 1139 (1980) describe II-trans-leukotriene C.

According to the invention there is provided compounds which, in free acid form, are of formula Ia — Ig,

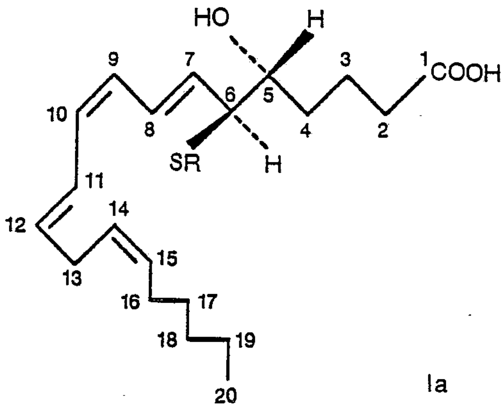

Ia

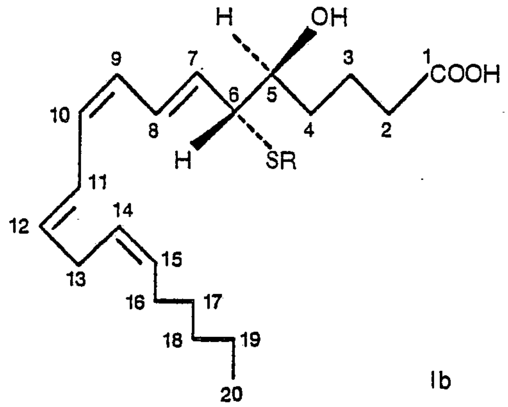

Ib

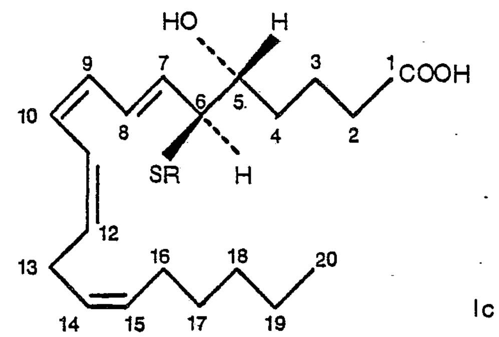

Ic

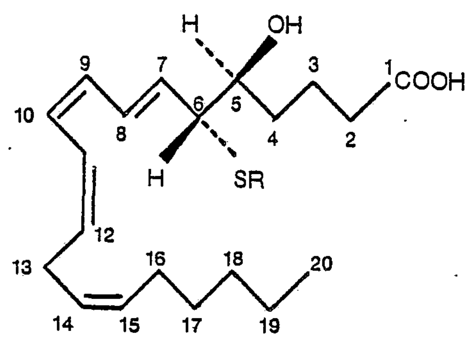

Id

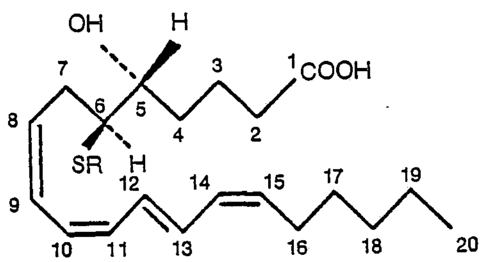

Ie

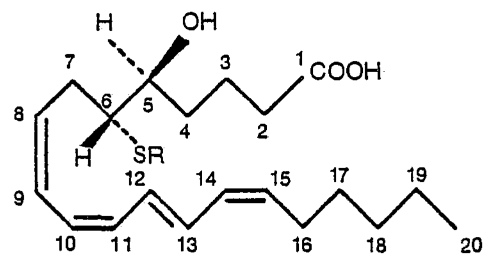

If

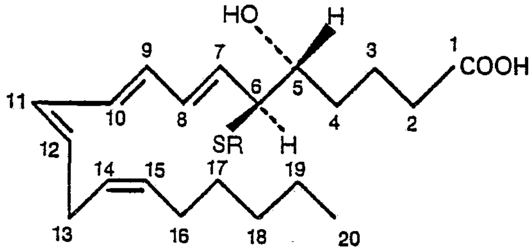

Ig

in which R, in free acid form is

$$-CH_2-\underset{\displaystyle NHR_2}{\underset{|}{CH}}CO-R_1,$$

wherein (C) $R_1$ is $NHCH_2COOH$
and $R_2$ is

$$COCH_2CH_2\underset{\displaystyle NH_2}{\underset{|}{CH}}-COOH$$

or (D) $R_1$ is $NHCH_2COOH$
and $R_2$ is H,
or (E) $R_1$ is OH
and $R_2$ is H.

It will be appreciated that, of the above compounds, those of formulae Ia and Ib, Ic and Id and Ie and If are enantiomeric pairs, differing from each other in their chirality at $C_5$ and $C_6$. The compounds of formulae Ia, Ic, Ie (and Ig) have the chirality 5R, 6S and the compounds of formula Ib, Id and If have the chirality 5S, 6R.

The compounds of formulae Ia and Ib have the double bond configuration 7E, 9Z, 11E, 14Z. The compounds of formulae Ic and Id have the double bond configuration 7E, 9Z, 11Z, 14Z. The compounds of formulae Ie and If have the double bond configuration 8Z, 10Z, 12E, 14Z and the compounds of formulae Ig have the double bond configuration 7E, 9E, 11E, 14Z.

In the above formulae, the compounds, for convenience, are shown in free acid form. They exist also in lactone, salt and ester forms, which forms are also embraced by the present invention. When in lactone form, their partial formulae, as will be appreciated, are as follows

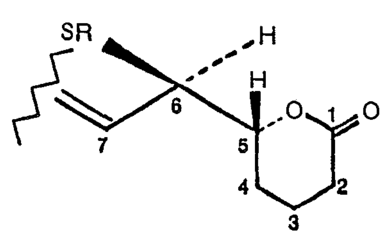

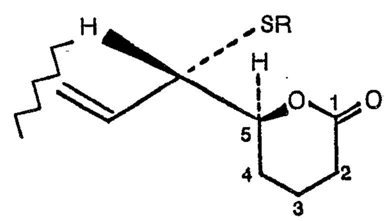

Of the salt forms, the pharmaceutically acceptable salt forms are preferred and as examples may be given the alkali and alkaline earth metal salt forms as well as ammonium and amine salt forms, the sodium and potassium salt forms being particularly preferred.

Of the ester forms, the pharmaceutically acceptable ester forms are preferred and as examples may be given the alkyl, silyl, cycloalkyl, cycloalkyl-alkyl and aralkyl, the $C_{1-4}$ alkyl esters being preferred.

Since the compounds of the invention contain two or three carboxylic acid functions, depending on the significance of $R_1$ and $R_2$, so called partial salts and partial esters, i.e. compounds in which not all the carboxylic acid functions are in salt or ester form, are possible.

The invention also provides a process for the production of the compounds of the invention, which process comprises

(a) obtaining a compound which, in free acid form, is of formula Ia or Ib, above, by reacting a compound which, in free acid form, is of formula II,

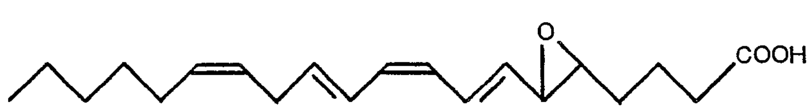

II

with a compound of formula III,

$$HS-CH_2-\underset{\displaystyle NHR_2}{\underset{|}{CH}}\ COR_1 \qquad (III)$$

in which $R_1$ and $R_2$ are as defined above, optionally in protected form,

(b) obtaining a compound which, in free acid form, is of formula Ic or Id, above, by reacting a compound which, in free acid form, is of formula IV

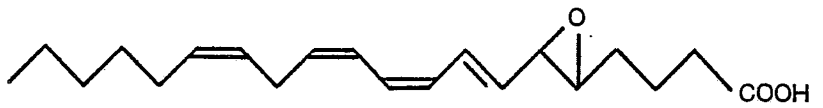

IV

with a compound of formula (III), above, optionally in protected form

(c) obtaining a compound which, in free acid form, is of formula Ig, above, by reacting a compound which, in free acid form, is of formula V,

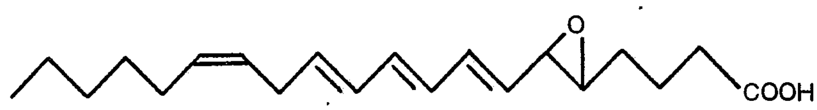

V

with a compound of formula III, above, optionally in protected form, or (d) obtaining a compound which, in free acid form, is of formula Ie or If, above, by double bond rearrangement (1,7 hydride shift) of a compound which, in free acid form, is of formula Ic or Id, the above reactions being followed, where required, by removal of any protecting group(s) and, where desired, by isolation of the resulting compounds in free acid, lactone, salt or ester form and, where desired, by separation of the enantiomeric 5R, 6S and 5S, 6R pairs.

The above reactions a) to c) are suitably carried out in the presence of a strong base (pKa>12) such as trialkylamine, e.g. triethylamine, and in an inert polar solvent such as in an alkanol, e.g. methanol. A suitable reaction temperature is from 10° to 50°C, preferably room temperature. Reaction times are generally of the order of from 15 minutes to 3 to 4 hours. In the above reactions it is preferred for the compounds of formulae II, IV and V to be employed in ester form, particularly in $C_{1-4}$ alkyl ester form and especially in the methyl ester form.

The 1,7 hydride shift involved in the double bond rearrangement d) is temperature and time dependent occuring already at room temperature when compounds of formula Ic and Id are dissolved in a solvent, and progressing with lapse of time. At higher temperatures the rearrangement is accelerated. It will, therefore, be appreciated that when carrying out reaction b), and it is desired to obtain a high yield of compounds of formula Ic or Id, it is preferred to employ relatively low reaction temperatures and/or relatively short reaction times. On the other hand, where it is desired to obtain compounds of formula Ie or If, reactions b) and d) can effectively be combined by employing, in reaction b), relatively high reaction temperatures and/or relatively long reaction times, *in situ* double bond rearrangement d) then occuring to yield compounds of formula Ie or If.

The above reactions a) to c) employ a racemic E epoxide and, accordingly, the products resulting comprise mixtures of both the 5R,6S and 5S,6R enantiomers. Where it is desired to separate the pair, this may be carried out in conventional manner. Where, however, in the compounds of the invention, $R_1$ and $R_2$ have the significances (C), above, a preferred method is the use of reverse phase HPLC on the compounds in free acid form. Where, however, $R_1$ and $R_2$ have the significances (D) or (E), above, the separation is preferably carried out using conventional HPLC methods on the compounds when in ester form.

It will be appreciated that the radical R, in the compounds of the invention, contains at least one optically active centre (two when $R_1$ and $R_2$ have significance (C)). It is not intended that the present invention be limited to any particular isomeric form of compounds arising from such centre(s).

Interconversion as between the various forms of the compounds of the invention, e.g. salt, free acid, lactone and ester forms may be carried out in conventional manner. For example, ester forms can be converted into salt forms by treatment with the appropriate aqueous dilute base at a pH of from 9 to 10. The salt forms can be converted to the free acid forms by aqueous acidification. The free acid forms can be converted to the lactone forms by acid treatment at a pH of less than 5, and the salt or acid forms can be converted to ester forms by base or acid catalysed esterification using an appropriate alcohol.

The compounds of formulae II, III, IV and V used in the process of the invention are either known or may be obtained in conventional manner from available starting materials. The compound of formula III, wherein $R_1$ and $R_2$ have the significance (D), above, is preferably employed in protected form, it being particularly preferable to use N-trifluoroacetylcysteinylglycine methyl ester. Deprotection in the final compound can be carried out in conventional manner, e.g. by base hydrolysis.

Representative compounds of the invention are as follows

a) representative of the compounds of formula Ia:—

5(R)-Hydroxy-6(S)-S-glutathionyl-7E,9Z,11E,14Z-eicosatetraenoic acid,

5(R)-Hydroxy-6(S)-S-cysteinylglycinyl-7E,9Z,11E,14Z-eicosatetraenoic acid and

5(R)-Hydroxy-6(S)-S-cysteinyl-7E,9Z,11E,14Z-eicosatetraenoic acid;

b) representative of the compounds of formula Ib:—
5(S)-Hydroxy-6(R)-S-glutathionyl-7E,9Z,11E,14Z-eicosatetraencic acid,
5(S)-Hydroxy-6(R)-S-cysteinylglycinyl-7E,9Z,11E,14Z-eicosatetraenoic acid and
5(S)-Hydroxy-6(R)-S-cysteinyl-7E,9Z,11E,14Z-eicosatetraenoic acid;

c) representative of the compounds of formula Ic:—
5(R)-Hydroxy-6(S)-S-glutathionyl-7E,9,11,14Z-eicosatetraenoic acid,
5(R)-Hydroxy-6(S)-S-cysteinylglycinyl-7E,9,11,14Z-eicosatetraenoic acid and
5(R)-Hydroxy-6(S)-S-cysteinyl-7E,9,11,14Z-eicosatetraenoic acid;

d) representative of the compounds of formula Id:—
5(S)-Hydroxy-6(R)-S-glutathionyl-7E,9,11,14Z, eicosatetraenoic acid,
5(S)-Hydroxy-6(R)-S-cysteinylglycinyl-7E,9,11,14Z-eicosatetraenoic acid and
5(S)-Hydroxy-6(R)-S-cysteinyl-7E,9,11,14Z-eicosatetraenoic acid;

e) representative of the compounds of formula Ie:—
5(R)-Hydroxy-6(S)-S-glutathionyl-8,10Z,12E,14Z-eicosatetraenoic acid
5(R)-Hydroxy-6(S)-S-cysteinylglycinyl-8,10Z,12E,14Z-eicosatetraenoic acid
5(R)-Hydroxy-6(S)-S-cysteinyl-8,10Z,12E,14Z-eicosatetraenoic acid;

f) representative of the compounds of formula If:—
5(S)-Hydroxy-6(R)-S-glutathionyl-8,10Z,12E,14Z-eicosatetraenoic acid,
5(S)-Hydroxy-6(R)-S-cysteinylglycinyl-8,10Z,12E,14Z-eicosatetraenoic acid and
5(S)-Hydroxy-6(R)-S-cysteinyl-8,10Z,12E,14Z-eicosatetraenoic acid,

g) representative of the compounds of formula Ig:—
5(R)-Hydroxy-6(S)-S-glutathionyl-7,9,11E,14Z-eicosatetraenoic acid,
5(R)-Hydroxy-6(S)-S-cysteinylglycinyl-7,9,11E,14Z-eicosatetraenoic acid,
5(R)-Hydroxy-6(S)-S-cysteinyl-7,9,11E,14Z-eicosatetraenoic acid.

The compounds of the present invention are pharmacologically active, being SRS—A partialagonists/antagonists as indicated by tests *in vitro* on guinea pig ileum segments at concentrations of from 0.1 ng to 1 µg according to the method of Schild 1947 Brit. J. Pharm. 2 197—206 and the *in vivo* Guinea Pig Pulmonary Function Test of Austen and Drazen 1974 J. Clin. Invest 53: 1679—1685 at dosages of from 0.1 µg/Kg to 0.1 mg/Kg. The compounds are accordingly indicated for therapeutic use in the treatment of allergic/anti-inflammatory reactions of the pulmonary system where SRS—A is thought to be a casual mediator, i.e. in allergic lung disorders such as extrinsic asthma and industrial asthmas such as Farmers lung and Pigeon Fanciers lung, bronchitis and cystic fibrosis, as well as in other disorders involving SRS—A as casual mediator, such as allergic skin diseases, ectopic and atopic eczemas, contact hypersensitivity and angioneurotic oedema. Compounds of the invention are also indicated for use in the treatment of hypertensive or hypotensive conditions as shown by their effect on systemic blood pressure in unconscious rats, according to the test described in Pharmacological Experiments on Intact Preparations, University of Edinburgh (1970)63.

The compounds may be administered by various routes. Thus, they may be administered by the oral and rectal routes, topically and parenterally e.g. by injection, being usually employed in the form of a pharmaceutical composition. Such compositions form part of the present invention and are prepared in a manner well known in the pharmaceutical art and normally comprise at least one compound of the invention in association with a pharmaceutically-acceptable carrier therefor. In making the compositions of the present invention, the active ingredient will usually be mixed with a carrier, or diluted by a carrier, or enclosed within a carrier which may be in the form of a capsule, sachet, paper or other container. When the carrier serves as a diluent, it may be a solid, semi-solid or liquid material which acts as a vehicle, excipient or medium for the active ingredient. Thus the composition can be in the form of tablets, lozenges, sachets, cachets, elixirs, suspensions, aerosols (as a solid or in a liquid medium), ointments containing for example up to 10% by weight of the active compound, soft and hard gelatin capsules, suppositories, injection suspensions and sterile packaged powders.

Some examples of uuitable carriers are lactose, dextrose, sucrose sorbitol, mannitol, starches, gum acacia, calcium phosphate, alginates, tragacanth, gelatin, syrup, methyl cellulose, methyl- and propylhydroxybenzoate, talc, magnesium stearate or mineral oil. The compositions of the invention may, as is well known in the art, be formulated so as to provide quick, sustained or delayed release of the active ingredient after administration to the patient.

Preferably the compositions are formulated in a unit dosage form, each dosage containing from 5 µg to 10 mg, more usually 10 µg to 1 mg, of the active ingredient. The term "unit dosage form" refers to physically discrete units suitable as unitary dosages for human subjects and animals each unit containing a predetermined quantity of active material calculated to produce the desired therapeutic effect, in association with the required pharmaceutical carrier.

The active compounds are effective over a wide dosage range and for example dosages per day will normally fall within the range of 5 μg to 10 mg in the treatment of adult humans, more usually in the range of from 10 μg to 1 mg. However it will be understood that the amount of the compound actually administered will be determined by a physician, in the light of the relevant circumstances including the condition to be treated, the choice of compound to be administered and the chosen route of administration and therefore the above dosage ranges are not intended to limit the scope of the invention in any way.

The following Examples illustrate the invention.

Example 1

5(R)-Hydroxy-6(S)-S-cysteinylglycinyl-7E,9Z,11E,14Z-eicosatetraenoic acid and 5(S)-Hydroxy-6(R)-S-cysteinylglycinyl-7E,9Z,11E,14Z-eicosatetranoic acid (compounds of formulae Ia and Ib, respectively)

N-trifluoroacetylcysteinylglycine methyl ester (1.44 mg) (Tet. Lett. *21.* 3143, (1980)) was dissolved in dry methanol (50 μl) and triethylamine (1.0 mg) added. This solution was then added to methyl E 5,6 oxido-7E,9Z,11E,14Z-eicosatetraenoate (0.8 mg) (Tet. Lett. *21*, 4123, (1980)) and the resultant solution allowed to stand at room temperature form 3 hours. The 5R, 6S and 5S and 6R isomers of the dimethyl N-trifluoroacetyl derivatives of the title compounds so formed were then separated by high pressure chromatography using a column packed with S5NH Spherisorb silica eluted with dichlormethane plus 0.5% methanol. The isomers eluted in the order 5R, 6S first 5S, 6R second, both compounds being colourless oils, λ max. 269,278 and 291 nm.

The isomers were separately hydrolysed at room temperature overnight using 3 parts aqueous 0.1M potassium carbonate solution to 1 part methanol. The title compounds were then further purified by high pressure chromatography using a column packed with 5 μ $C_{18}$ Nucleosil reverse phase silica eluted with methanol: water 70:30, the pH being adjusted to 5.2 with acetic acid.

Example 2

5(R)-Hydroxy-6(S)-S-cysteinylglycinyl 7E,9,11,14Z-eicosatetraenoic acid and 5(S)-Hydroxy-6(R)-S-cysteinylglycinyl 7E,9,11,14Z-eicosatetraenoic acid (compounds of formulae Ic and Id, respectively)

The title compounds were prepared from methyl E 5,6 oxido-7E,9,11,14Z eicosatetraenoate (Tet. Lett. *21*, 4123, (1980)) using the procedure given in Example 1; λ max. 271, 281 and 291 nm.

Example 3

5(R)-Hydroxy-6(S)-cysteinylglycinyl 7,9,11E,14Z-eicosatetraenoic acid

The title compound was prepared from methyl E 5,6 oxide-7,9,11E,14Z-eicosatetraenoate (Tet. Lett. *21*, 3143 (1980)) using the procedure given in Example 1, above; λ max. 276, 277 and 287 nm.

Example 4

5(R)-Hydroxy-6(S)-S-cysteinylglycinyl 8,10Z,12E,14Z-eicosatetraenoic acid and 5(S)-Hydroxy-6(R)-S-cysteinylglycinyl 8,10Z,12E,14Z-eicosatetraenoic acid (compounds of formulae Ie and If, respectively).

The title compounds were obtained as minor products when applying the procedure described in Example 2. They were both isolated as colourless oils, λ max. 281, 293, 306 and 322 nm, using the reverse phase high pressure chromatography system described in Example 1.

Example 5

5(R)-Hydroxy-6(S)-S-glutathionyl-7E,9Z,11E,14Z-eicosatetraenoic acid and 5(S)-Hydroxy-6(R)-S-glutathionyl-7E,9Z,11E,14Z-eicosatetraenoic acid (compounds of formula Ia and Ib, respectively).

Triethylamine (6.06 mg) was added to glutathione (4.6 mg) and the mixture dissolved in dry methanol (50 μl). This solution was then added to methyl E 5,6 oxido-7E,9Z,11E,14Z-eicosatetraenoate (1.66 mg) (Tet. Lett. *21*, 4123, (1980)) and the resultant solution allowed to stand at room temperature for 3 hours. The reaction mixture was then evaporated to dryness to give methyl 5(RS)-Hydroxy-6(SR)-S-glutathionyl-7E,9Z-11E-14Z-eicosatetraenoate was redissolved in 3 parts aqueous 0.1 M potassium carbonate solution to 1 part methanol and the resultant clear solution allowed to stand at room temperature overnight. The title compounds were then separated using high pressure chromatography using a column packed with 5 μ $C_{18}$ Nucleosil reverse phase silica eluted with methanol/water (60:40) adjusted to a pH of 4.2 wtih acetic acid. The order of elution using this system is first 5R, 6S isomer, second 5S,6R isomer, both with λ max. 269, 278 and 291 nm.

Example 6

5(R)-Hydroxy-6(S)-S-glutathionyl-7E,9,11,14Z-eicosatetraenoic acid and 5(S)-Hydroxy-6(R)-S-glutathionyl-7E,9,11,14Z-eicosatetraenoic acid (compounds of formulae Ic and Id, respectively).

The title compounds were prepared from methyl E 5,6 oxido-7E,9,11,14Z-eicosatetraenoate (Tet. Lett. *21*, 4123 (1980)) using the procedure given in Example 5; λ max. 271, 281, and 291 nm.

Example 7

5(R)-Hydroxy-6(S)-S-glutathionyl-7,9,11E,14Z-eicosatetraenoic acid (compound of formula Ig)

The title compound was prepared from methyl E 5,6 oxido-7,9,11E,14Z-eicosatetraenoate (Tet. Lett. *21* 3143 (1980)) using the procedure given in Example 5; λ max. 267, 277 and 287 nm.

Example 8

5(R)-Hydroxy-6(S)-S-cysteinyl-7E,9Z,11E,14Z-eicosatetraenoic acid and
5(S)-Hydroxy-6(R)-S-cysteinyl-7E,9Z,11E,14Z-eicosatetraenoic acid (compounds of formulae Ia and Ib, respectively)

N-trifluoroacetylcysteine methyl ester (1.23 mg) was dissolved in dry methanol (50 μl) and triethylamine (1.0 mg) added. This solution was then added to methyl E 5,6-oxido-7E,9Z,11E,14Z-eicosatetraenoate (0.8 mg) (Tet. Lett. *21*, 4123, (1980)) and the resultant solution allowed to stand at room temperatures for 20 minutes. The 5R,6S and 5S,6R isomers of the dimethyl N-trifluoroacetyl derivatives of the title compounds so formed were then separated by high pressure chromatography using a column packed with S5NH Spherisorb eluted with Hexane:dichloromethane:methanol 75:25:1. The isomers were isolated as colourless oils; λ max. 269, 278 and 291 nm.

The isomers were separately hydrolyzed at room temperature overnight using 3 parts aqueous 0.1 m potassium carbonate solution to 1 part methanol. The title compounds were then further purified by high pressure chromatography using a column packed with 5 μ $C_{18}$ Nucleosil reverse phase silica eluted with methanol: water 70:30, the pH being adjusted to 5.2 with acetic acid.

Example 9

5(R)-Hydroxy-6(S)-S-cysteinyl-7E,9,11,14Z-eicosatetraenoic acid and
5(S)-Hydroxy-6(R)-S-cysteinyl-7E,9,11,14Z-eicosatetraenoic acid (compounds of formulae Ic and Id, respectively)

The title compounds were prepared from methyl E 5,6 oxido-7E,9,11,14Z-eicosatetraenoate (Tet. Lett. *21*, 4123 (1980)) using the procedure given in Example 8; λ max. 271, 281 and 291 nm.

Example 10

5(R)-Hydroxy-6(S)-S-cysteinyl-7,9,11E,14Z-eicosatetraenoic acid (compound of formula Ig)

The title compound was prepared from methyl E 5,6 oxido-7,9,11E,14Z eicosatetraenoate (Tet. Lett. *21* 3143 (1980)) using the procedure given in Example 8; λ max. 267, 277 287 nm.

Example 11

5(R)-Hydroxy 6(S)-S-glutathionyl 8,10Z,12E,14Z-eicosatetraenoic acid and
5(S)-Hydroxy 6(R)-S-glutathionyl 8,10Z,12E,14Z-eicosatetraenoic acid (compounds of formulae Ie and If, respectively)

The title compounds were obtained as minor products when applying the procedure described in Example 6. They were both isolated as colourless oils, λ max. 281, 293, 306 and 322 nm, using the reverse phase high pressure chromatography system described in Example 5.

**Claims for the Contracting States: BE CH DE FR GB IT LI LU NL SE**

1. A compound which, in free acid form, is one of the formulae Ia—Ig.

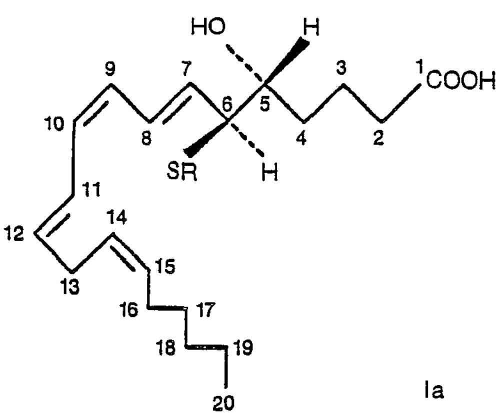

Ia

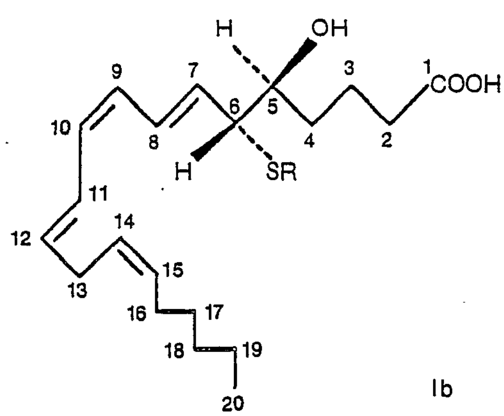

Ib

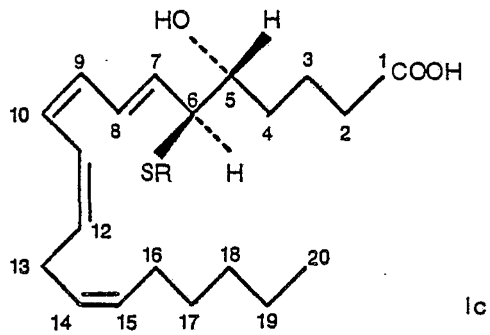

Ic

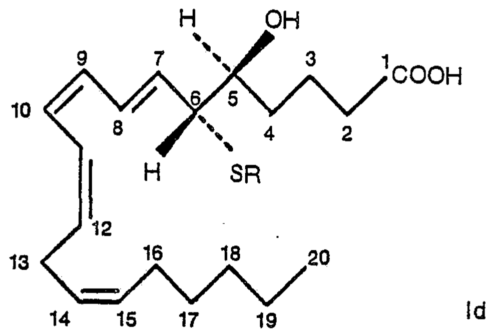

Id

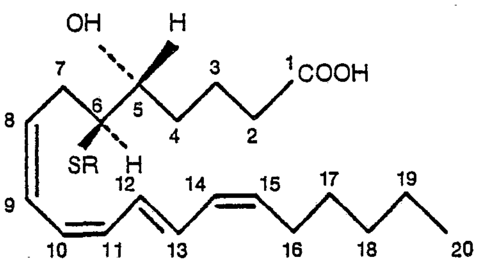

Ie

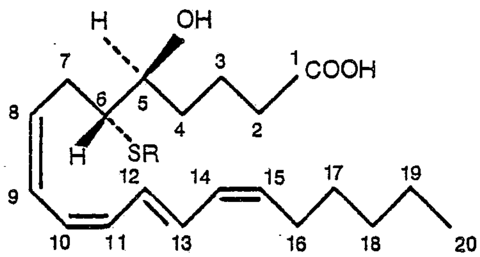

If

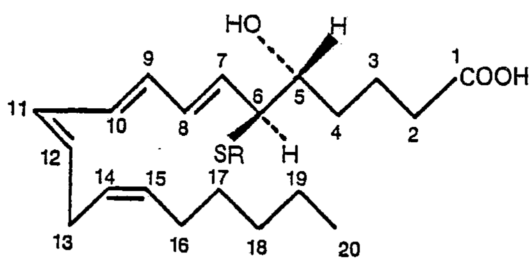

Ig

in which R, in free acid form is

$$-CH_2-\underset{\displaystyle NHR_2}{\underset{|}{CH}}CO-R_1,$$

wherein (C) $R_1$ is $NHCH_2COOH$
and $R_2$ is

$$COCH_2CH_2\underset{\displaystyle NH_2}{\underset{|}{CH}}-COOH$$

or (D) $R_1$ is $NHCH_2COOH$
and $R_2$ is H,
or (E) $R_1$ is OH
and $R_2$ is H.

2. A compound of claim 1 in free acid form.
3. A compound of claim 1 in lactone form.
4. A compound of claim 1 in ester form.
5. A compound of claim 4 in pharmaceutically acceptable ester form.
6. A compound of claim 1 in salt form.
7. A compound of claim 6 in pharmaceutically acceptable salt form.
8. A process for the production of a compound of claim 1 which comprises:

(a) obtaining a compound which, in free acid form, is of formula Ia or Ib, above, by reacting a compound which, in free acid form, is of formula II,

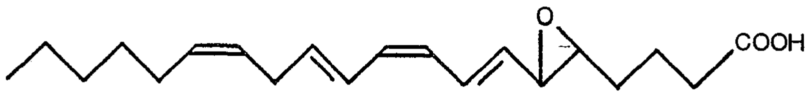

II

with a compound of formula III,

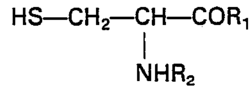

(III)

in which $R_1$ and $R_2$ are as defined above, optionally in protected form,

(b) obtaining a compound which, in free acid form, is of formula Ic or Id, above, by reacting a compound which, in free acid form, is of formula IV

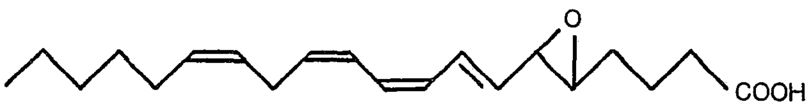

IV

with a compound of formula (III), above, optionally in protected form

(c) obtaining a compound which, in free acid form, is of formula Ig, above, by reacting a compound which, in free acid form, is of formula V,

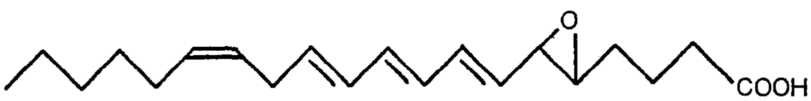

V

with a compound of formula III, above, optionally in protected form, or

(d) obtaining a compound which, in free acid form, is of formula Ie or If, above, by double bond rearrangement (1,7 hydride shift) of a compound which, in free acid form, is of formula Ic or Id, the above reactions being followed, where required, by removal of any protecting group(s) and, where desired, by isolation of the resulting compounds in free acid, lactone, salt or ester form and, where desired, by separation of the enantiomeric 5R,6S and 5S,6R pairs.

9. A pharmaceutical composition comprising a compound of claim 1 in free acid, lactone or pharmaceutically acceptable salt or ester form, in association with a pharmaceutically acceptable diluent or carrier.

10. A compound of claim 1 for use in the treatment of allergic/inflammatory disorders involving SRS—A as casual mediator.

**Claims for the Contracting State AT**

1. A process for the production of a compound which is one of the formulae Ia—Ig.

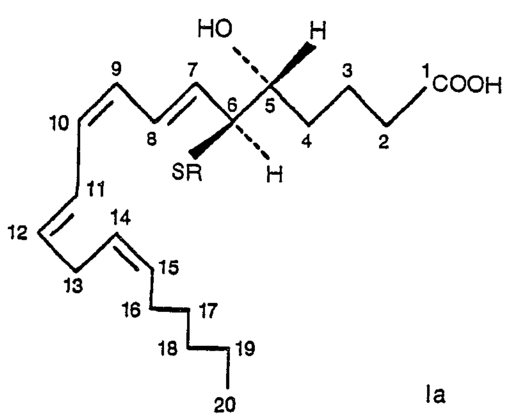

Ia

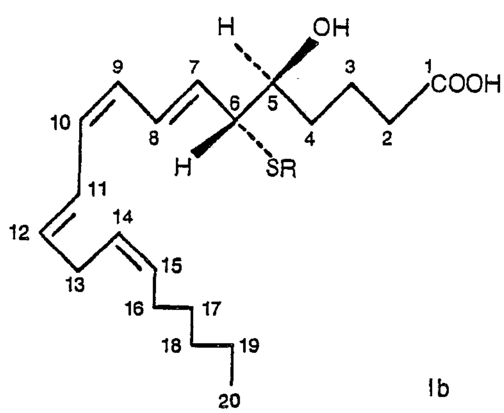

Ib

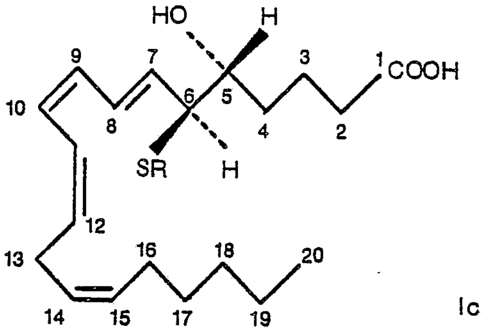

Ic

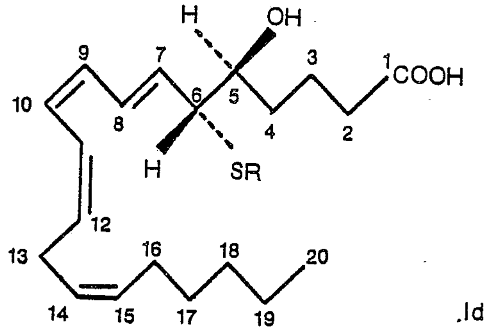

Id

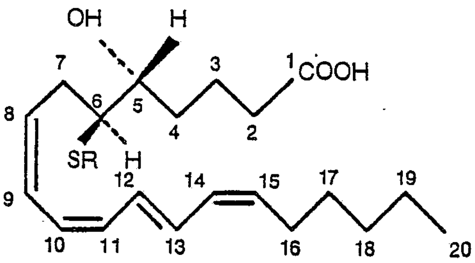

Ie

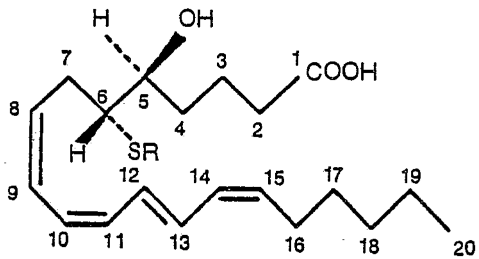

If

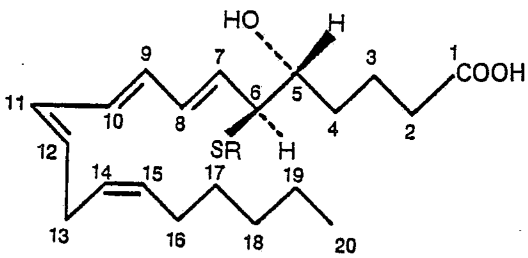

Ig

which comprises:

(a) obtaining a compound which, in free acid form, is of formula Ia or Ib, above, by reacting a compound which, in free acid form, is of formula II,

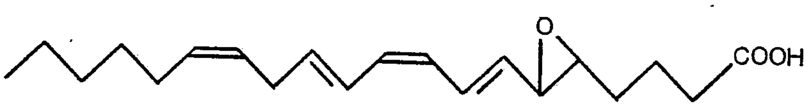

II

with a compound of formula III,

$$HS—CH_2—\underset{\displaystyle NHR_2}{\underset{|}{C}H}\ COR_1 \qquad (III)$$

in which $R_1$ and $R_2$ are as defined above, optionally in protected form,

(b) obtaining a compound which, in free acid form, is of formula Ic or Id, above, by reacting a compound which, in free acid form, is of formula IV

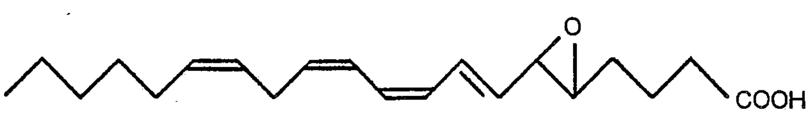

IV

with a compound of formula (III), above, optionally in protected form

(c) obtaining a compound which, in free acid form, is of formula Ig, above, by reacting a compound which, in free acid form, is of formula V,

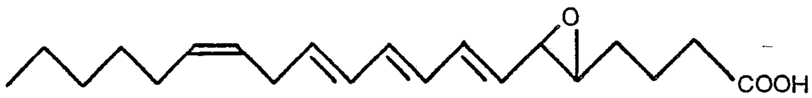

V

with a compound of formula III, above, optionally in protected form, or

(d) obtaining a compound which, in free acid form, is of formula Ie or If, above, by double bond rearrangement (1,7 hydride shift) of a compound which, in free acid form, is of formula Ic or Id, the above reactions being followed, where required, by removal of any protecting group(s) and, where desired, by isolation of the resulting compounds in free acid, lactone, salt or ester form and, where desird, by separation of the enantiomeric 5R,6S and 5S,6R pairs.

2. A process according to claim 1 for the production of a compound as defined in claim 1 in free acid form.

3. A process according to claim 1 for the production of a compound of claim 1 in lactone form.

4. A process according to claim 1 for the production of a compound of claim 1 in ester form.

5. A process according to claim 1 for the production of a compound of claim 4 in pharmaceutically acceptable ester form.

6. A process according to claim 1 for the production of a compound of claim 1 in salt form.

7. A process according to claim 1 for the production of a compound of claim 6 in pharmaceutically acceptable salt form.

**Patentansprüche für die Vertragsstaaten BE CH DE FR GB IT LI LU NL SE:**

1. Verbindung, welche, in Form der freien Säure, eine der Formeln Ia bis Ig aufweist

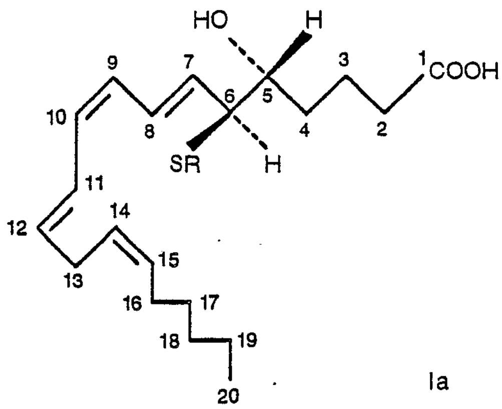

Ia

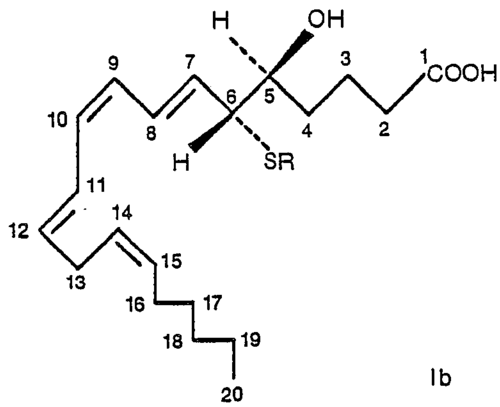

Ib

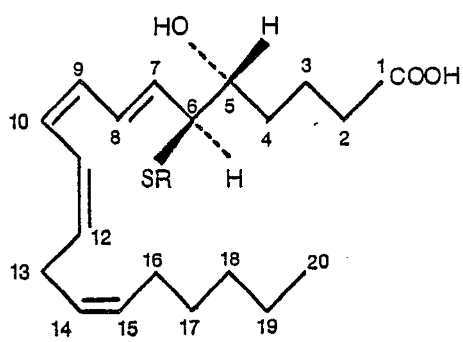

Ic

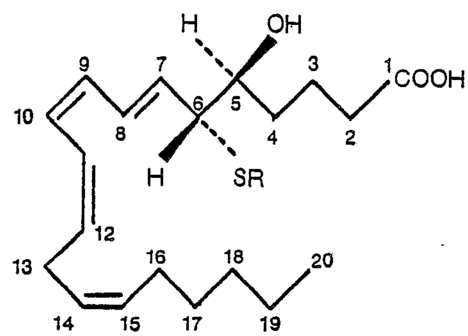

Id

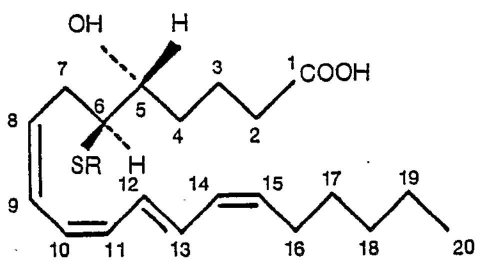

Ie

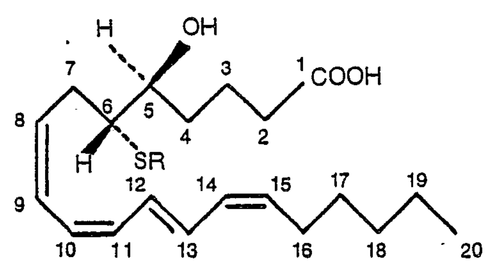

If

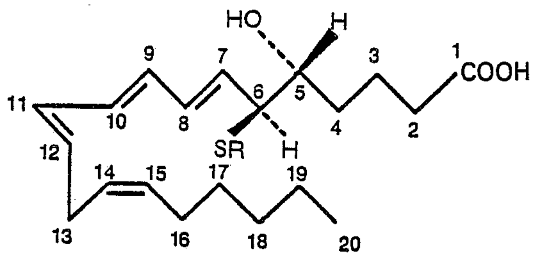

Ig

worin R in der freie-Säure-Form

$$-CH_2-\underset{\displaystyle NHR_2}{\underset{|}{C}}HCO-R_1$$

ist, worin (C) $R_1$ $NHCH_2COOH$ ist und $R_2$

$$COCH_2CH_2\underset{\displaystyle NH_2}{\underset{|}{C}}H-COOH$$

ist oder (D) $R_1$ $NHCH_2COOH$ ist und $R_2$ H ist oder (E) $R_1$ OH ist und $R_2$ H ist.

2. Verbindung nach Anspruch 1 in Form der freien Säure.

3. Verbindung nach Anspruch 1 in der Lactonform.

4. Verbindung nach Anspruch 1 in Form eines Esters.

5. Verbindung nach Anspruch 4 in Form eines pharmazeutisch annehmbaren Esters.

6. Verbindung nach Anspruch 1 in Form eines Salzes.

7. Verbindung nach Anspruch 6 in Form eines pharmazeutisch annehmbaren Salzes.

8. Verfahren zur Herstellung einer Verbindung gemäß Anspruch 1, welches umfaßt:

(a) die Gewinnung einer Verbindung, welche, in Form der freien Säure, oben aufgeführte Formel Ia oder Ib aufweist, durch Umsetzung einer Verbindung, welche, in Form der freien Säure, die Formel II aufweist

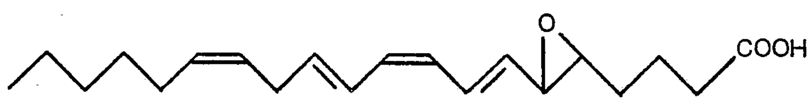

II

mit einer Verbindung der Formel III,

$$HS-CH_2-\underset{\displaystyle NHR_2}{\underset{|}{C}}H\ COR_1 \qquad (III)$$

worin $R_1$ und $R_2$ wie oben beschrieben definiert sind, gegebenenfalls in geschützter Form,

(b) Gewinnung einer Verbindung, welche, in Form der freien Säure, oben aufgeführte Formel Ic oder Id aufweist, durch Umsetzung einer Verbindung, welche, in Form der freien Säure, die Formel IV aufweist

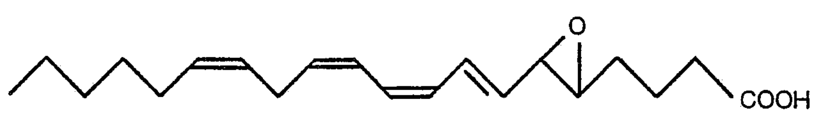

IV

mit einer Verbindung der oben aufgeführten Formel III, gegebenenfalls in geschützter Form,

(c) Gewinnung einer Verbindung, welche, in Form der freien Säure, oben erwähnte Formel Ig aufweist, durch Umsetzung einer Verbindung, welche, in Form der freien Säure, die Formel V aufweist

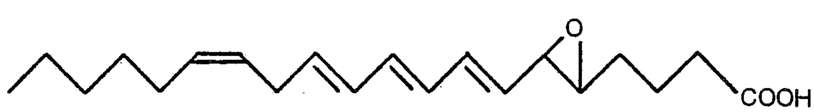

V

mit einer Verbindung der oben aufgeführten Formel III, gegebenenfalls in geschützter Form, oder

(d) Gewinnung einer Verbindung, welche, in Form der freien Säure, oben aufgeführte Formel Ie oder If

aufweist, durch Doppelbindungsumlagerung (1,7-Wasserstoffverschiebung) in einer Verbindung, welche, in Form der freien Säure, die Formel Ic oder Id aufweist, wobei auf oben erwähnte Umsetzungen, wenn nötig, die Abspaltung der Schutzgruppe(n) folgt und, falls erwünscht, die Isolierung der resultierenden Verbindungen in Form der freien Säure, des Lactons, eines Salzes, oder eines Esters und, falls erwünscht, die Trennung der Enantiomerenpaare 5R,6S und 5S,6R.

9. Pharmazeutisches Mittel, enthaltend eine Verbindung gemäß Anspruch 1 in Form der freien Säure, des Lactons oder eines pharmazeutisch annehmbaren Salzes oder Esters, in Verbindung mit einem pharmazeutisch annehmbaren Streckmittel oder Träger.

10. Verbindung gemäß Anspruch 1 zur Verwendung in der Behandlung von allgergischen/entzündlichen Störungen, bei denen SRS—A als vorübergehender Mittler beteiligt ist.

**Patentansprüche für den Vertragsstaat AT:**

1. Verfahren zur Herstellung einer Verbindung, welche eine der Formeln Ia bis Ig aufweist

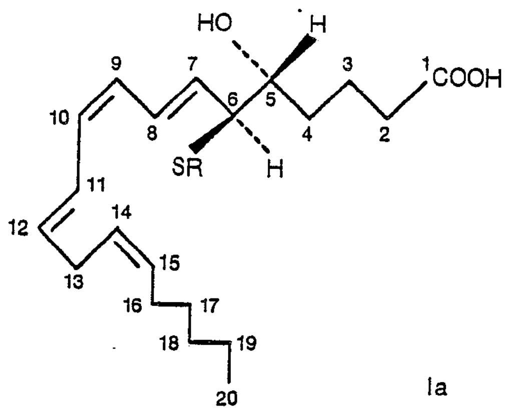
Ia

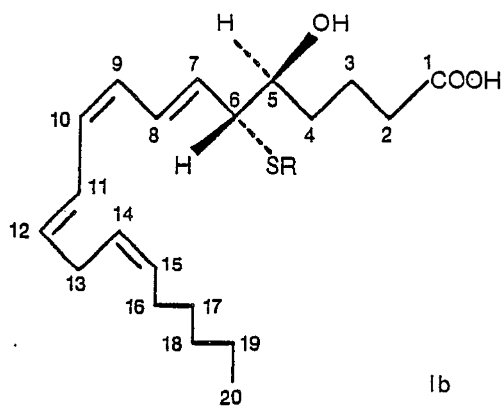
Ib

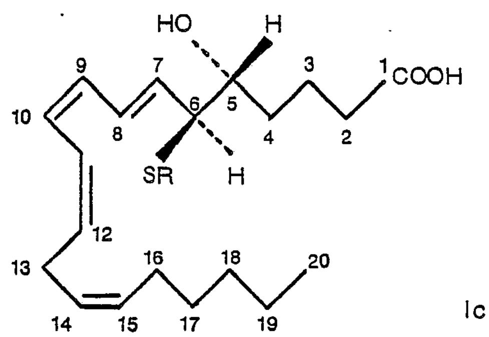
Ic

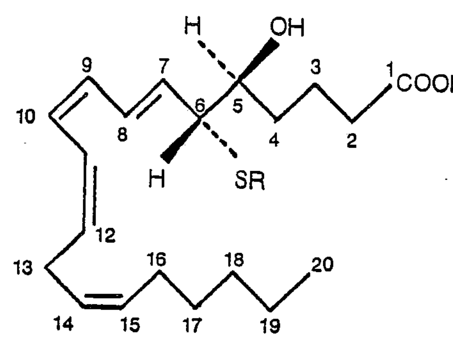
Id

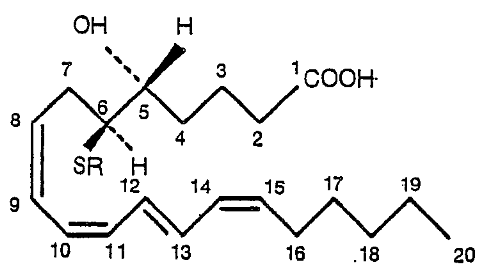
Ie

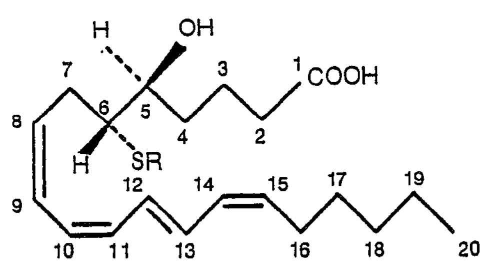
If

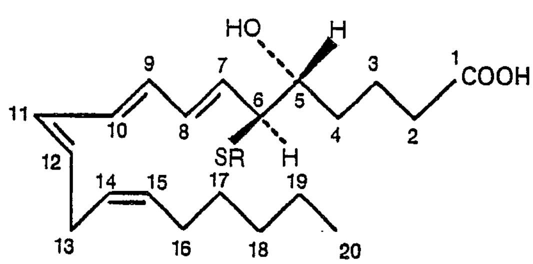
Ig

welches umfaßt:

(a) die Gewinnung einer Verbindung, welche, in Form der freien Säure, oben aufgeführte Formel Ia oder Ib aufweist, durch Umsetzung einer Verbindung, welche, in Form der freien Säure, die Formel II aufweist

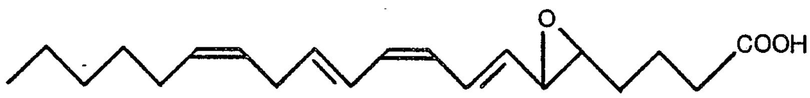

II

mit einer Verbindung der Formel III,

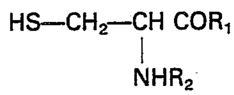

(III)

worin $R_1$ und $R_2$ wie oben beschreiben definiert sind, gegebenenfalls in geschützter Form,

(b) Gewinnung einer Verbindung, welche, in Form der freien Säure, oben aufgeführte Formel Ic oder Id aufweist, durch Umsetzung einer Verbindung, welche, in Form der freien Säure, die Formel IV aufweist

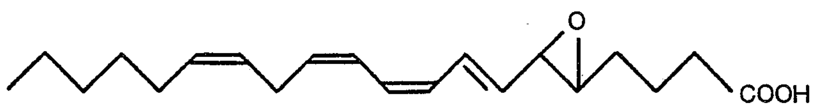

IV

mit einer Verbindung der oben aufgeführten Formel III, gegebenenfalls in geschützter Form,

(c) Gewinnung einer Verbindung, welche, in Form der freien Säure, oben erwähnte Formel Ig aufweist, durch Umsetzung einer Vebindung, welche, in Form der freien Säure, die Formel V aufweist

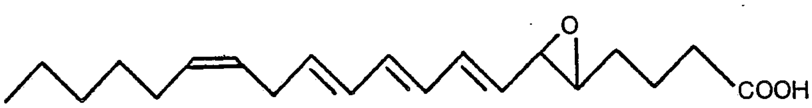

V

mit einer Verbindung der oben aufgeführten Formel III, gegebenenfalls in geschützter Form, oder

(d) Gewinnung einer Verbindung, welche in Form der freien Säure, oben aufgeführte Formel Ie oder If aufweist, durch Doppelbindungsumlagerung (1,7-Wasserstoffverschiebung) in einer Verbindung, welche, in Form der freien Säure, die Formel Ic oder Id aufweist, wobei auf oben erwähnte Umsetzungen, wenn nötig, die Abspaltung der Schutzgruppe(n) folgt und, falls erwünscht, die Isolierung der resultierenden Verbindungen in Form der freien Säure, des Lactons, eines Salzes, oder eines Esters und, falls erwünscht, die Trennung der Enantiomerenpaare 5R, 6S und 5S, 6R.

2. Verfahren nach Anspruch 1 zur Herstellung einer Verbindung, wie definiert in Anspruch 1, in Form der freien Säure.

3. Verfahren nach Anspruch 1 zur Herstellung einer Verbindung gemäß Anspruch 1 in Form des Lactons.

4. Verfahren nach Anspruch 1 zur Herstellung einer Verbindung gemäß Anspruch 1 in Form eines Esters.

5. Verfahren nach Anspruch 1 zur Herstellung einer Verbindung gemäß Anspruch 4 in Form eines pharmazeutisch annehmbaren Esters.

6. Verfahren nach Anspruch 1 zur Herstellung einer Verbindung gemäß Anspruch 1 in Form eines Salzes.

7. Verfahren nach Anspruch 1 zur Herstellung einer Verbindung gemäß Anspruch 6 in Form eines pharmazeutisch annehmbaren Salzes.

**Revendications pour les Etats contractants BE CH DE FR GB IT LI LU NL SE:**

1. Composé qui, sous forme de son acide libre, répond à une des formules Ia—Ig:

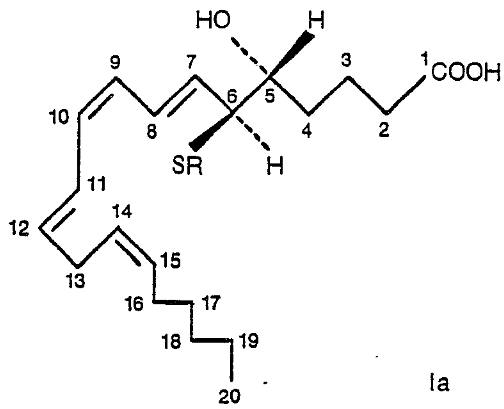

Ia

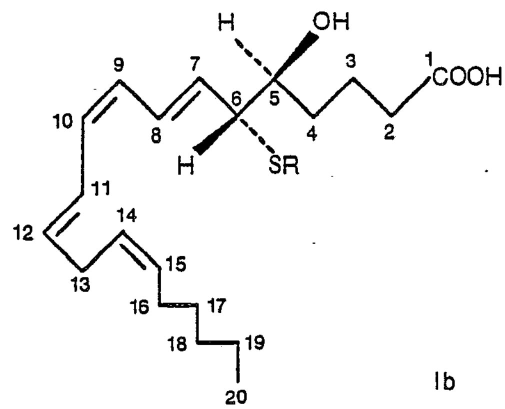

Ib

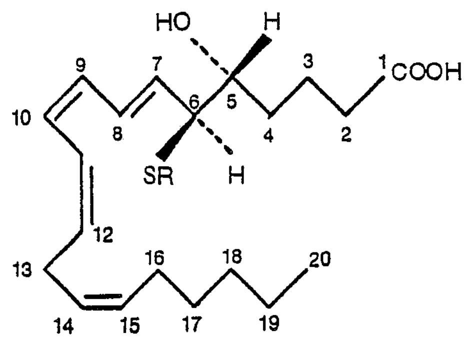

Ic

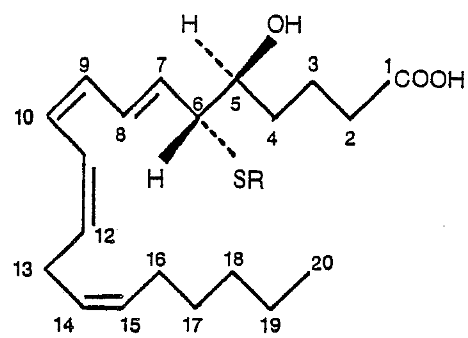

Id

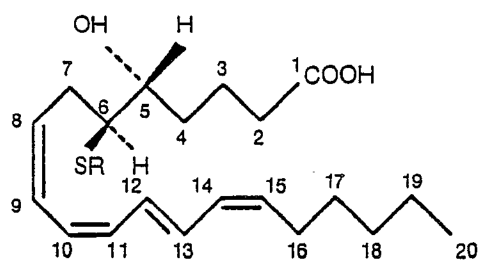

Ie

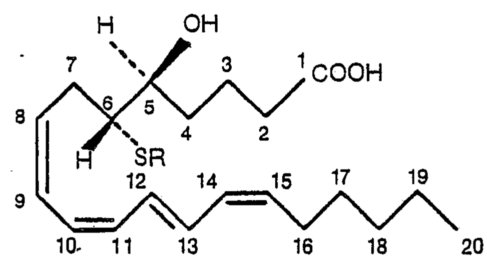

If

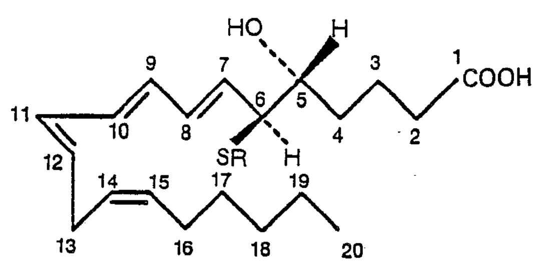

Ig

où R, sous forme d'acide libre, représente

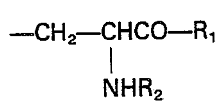

où (C) $R_1$ représente $NHCH_2COOH$ et
$R_2$ représente

$$COCH_2CH_2\underset{\displaystyle |\atop \displaystyle NH_2}{CH}—COOH$$

ou (D) $R_1$ représente $NHCH_2COOH$ et
$R_2$ représente H,
ou (E) $R_1$ représente OH et
$R_2$ représente H.

2. Composé suivant la revendication 1 sous forme d'un acide libre.
3. Composé suivant la revendication 1 sous forme d'une lactone.
4. Composé suivant la revendication 1 sous forme d'un ester.
5. Composé suivant la revendication 4 sous forme d'un ester pharmaceutiquement acceptable.
6. Composé suivant la revendication 1 sous forme d'un sel.
7. Composé suivant la revendication 6 sous forme d'un sel pharmaceutiquement acceptable.
8. Procédé pour la production d'un composé suivant la revendication 1, caractérisé en ce qu'il comprend les étapes qui consistent à:

(a) obtenir un composé qui, sous forme de son acide libre, répond à la formule Ia ou Ib ci-dessus, en faisant réagir un composé qui, sous forme de son acide libre, répond à la formule II:

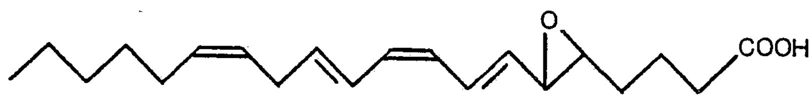

II

avec un composé de formule III:

$$HS—CH_2—\underset{\displaystyle |\atop \displaystyle NHR_2}{CH}\ COR_1 \qquad III$$

dans laquelle $R_1$ et $R_2$ ont les significations définies ci-dessus, éventuellement sous forme protégée,

(b) obtenir un composé qui, sous forme de son acide libre, répond à la formule Ic ou Id ci-dessus, en faisant réagir un composé qui, sous forme de son acide libre, répond à la formule IV:

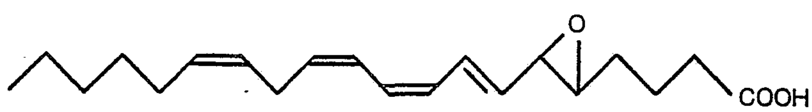

IV

avec un composé de formule (III) ci-dessus, éventuellement sous forme protégée,

(c) obtenir un composé qui, sous forme de son acide libre, répond à la formule Ig ci-dessus, en faisant réagir un composé qui, sous forme de son acide libre, répond à la formule V:

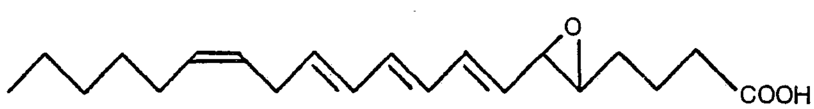

V

avec un composé de formule III ci-dessus, éventuellement sous forme protégée, ou

(d) obtenir un composé qui, sous forme de son acide libre, répond à la formule Ie ou If ci-dessus, par transposition à double liaison (décalage du 1,7-hydrure) d'un composé qui, sous forme de son acide libre, répond à la formule Ic ou Id, les réactions ci-dessus étant suivies, au besoin, de l'élimination de l'un ou l'autre groupe protecteur et, lorsqu'on le désire, de l'isolation des composés obtenus sous forme d'acides libres, de lactones, de sels ou d'esters et, lorsqu'on le désire, de la séparation des paires énantiomères 5R,6S et 5S,6R.

9. Composition pharmaceutique comprenant un composé suivant la revendication 1 sous forme d'un acide libre, d'une lactone, d'un ester ou d'un sel pharmaceutiquement acceptable, en association avec un diluant ou un support pharmaceutiquement acceptable.

10. Composé suivant la revendication 1, en vue de l'utiliser pour le traitement des troubles allergiques/inflammatoires impliquant SRS—A comme médiateur causal.

**Revendications pour l'Etat contractant AT:**

1. Procédé pour la production d'un composé répondant à une des formules Ia—Ig:

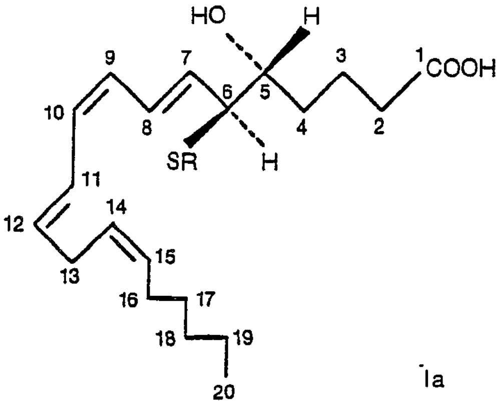

Ia

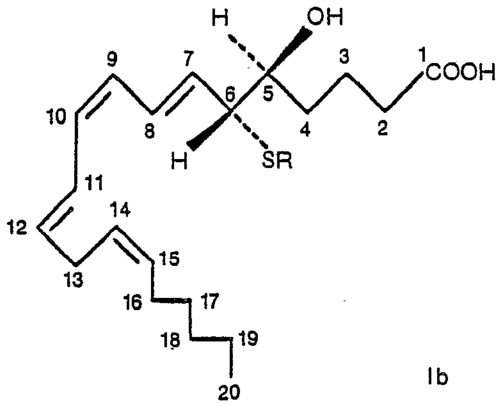

Ib

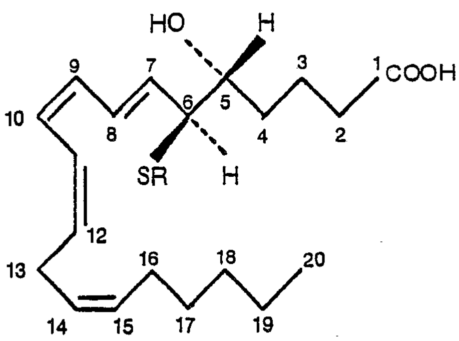

Ic

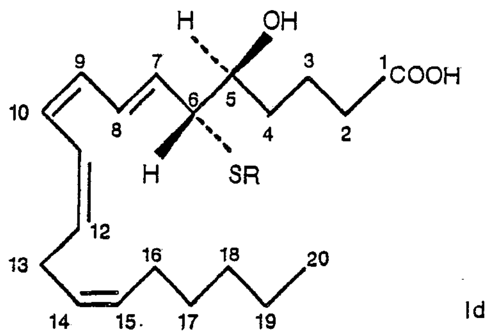

Id

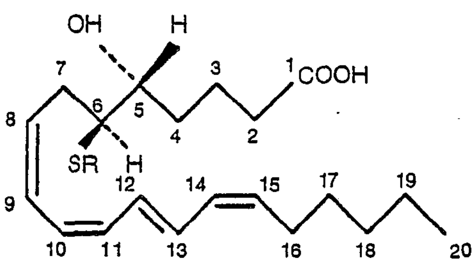

Ie

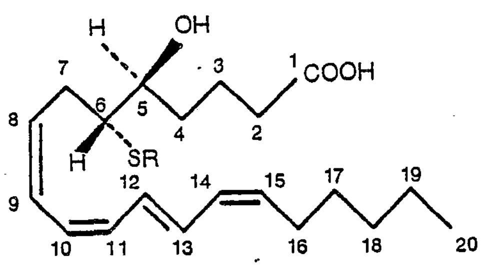

If

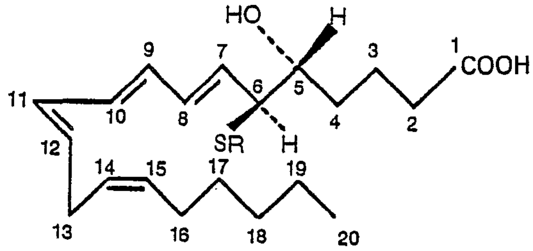

Ig

caractérisé en ce qu'il comprend les étapes qui consistent à:

(a) obtenir un composé qui, sous forme de son acide libre, répond à la formule Ia ou Ib ci-dessus, en faisant réagir un composé qui, sous forme de son acide libre, répond à la formule II:

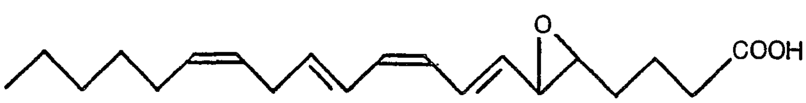

II

avec un composé de formule III:

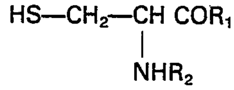

III

dans laquelle $R_1$ et $R_2$ ont les significations définies ci-dessus, éventuellement sous forme protégée,

(b) obtenir un composé qui, sous forme de son acide libre, répond à la formule Ic ou Id ci-dessus, en faisant réagir un composé qui, sous forme de son acide libre, répond à la formule IV:

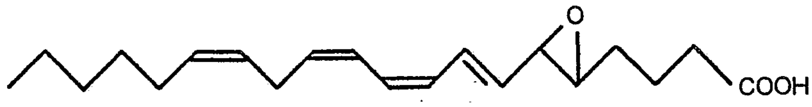

IV

avec un composé de formule (III) ci-dessus, éventuellement sous forme protégée,

(c) obtenir un composé qui, sous forme de son acide libre, répond à la formule Ig ci-dessus, en faisant réagir un composé qui, sous forme de son acide libre, répond à la formule V:

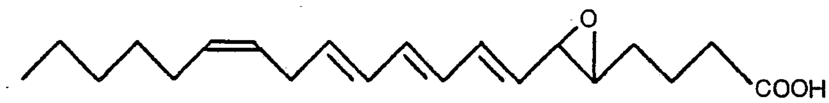

V

avec un composé de formule III ci-dessus, éventuellement sous forme protégée, ou

(d) obtenir un composé qui, sous forme de son acide libre, répond à la formule Ie ou If ci-dessus, par transposition à double liaison (décalage du 1,7-hydrure) d'un composé qui, sous forme de son acide libre répond à la formule Ic ou Id, les réactions ci-dessus étant suivies, lorsque cela est nécessaire, de l'élimination de l'un ou l'autre groupe protecteur et, lorsqu'on le désire, de l'isolation des composés obtenus sous forme d'acides libres, de lactones, de sels ou d'esters et, lorsqu'on le désire, de la séparation des paires énantiomères 5R,6S et 5S,6R.

2. Procédé suivant la revendication 1 pour la production d'un composé défini dans la revendication 1 sous forme d'un acide libre.

3. Procédé suivant la revendication 1 pour la production d'un composé de la revendication 1 sous forme d'une lactone.

4. Procédé suivant la revendication 1 pour la production d'un composé de la revendication 1 sous forme d'un ester.

5. Procédé suivant la revendiction 1 pour la production d'un composé de la revendication 4 sous forme d'un ester pharmaceutiquement acceptable.

6. Procédé suivant la revendication 1 pour la production d'un composé de la revendication 1 sous forme d'un sel.

7. Procédé suivant la revendication 1 pour la production d'un composé de la revendication 6 sous forme d'un sel pharmaceutiquement acceptable.